# EUROPEAN PATENT APPLICATION

(11) **EP 3 896 606 A1**
(43) Date of publication of application: **20.10.2021**
(21) Application number: 21164191.5
(22) Date of filing: 23.03.2021
(51) Int. Cl.: G06K 9/00, A61B 3/00

(54) **EXAMINATION TARGET CLASSIFICATION SYSTEM, EXAMINATION TARGET CLASSIFICATION METHOD, AND EXAMINATION TARGET CLASSIFICATION PROGRAM**

(30) Priority: 13.04.2020 JP 2020071450
(71) Applicant: Tomey Corporation, Nagoya-shi, Aichi 451-0051 (JP)
(72) Inventor: BIAN, Guangchun, Aichi-ken, 451-0051 (JP); YAMAGISHI, Koji, Aichi-ken, 451-0051 (JP); GOTO, Yoshito, Aichi-ken, 451-0051 (JP); TAKATA, Hideo, Aichi-ken, 451-0051 (JP)
(74) Representative: BSB Patentanwälte Schütte & Engelen Partnerschaft mbB

(57) **Abstract**

Provided is a technology that enables objective classification.

An examination target classification system includes an examination target image acquisition unit that acquires an examination target image which is an image of an examination portion of an anterior eye portion or a region around the anterior eye portion, a classification unit that inputs the examination target image to a machine learning model which classifies a state of the examination portion based on the image of the examination portion, and acquires a classification result, and a display unit that displays the classification result.

## Description

### Technical Field

The present invention relates to an examination target classification system, an examination target classification method, and an examination target classification program.

### Background Art

In the related art, a technology for evaluating an anterior eye portion or a region around the anterior eye portion as an examination target has been known. For example, Patent Literatures 1 and 2 disclose technologies for evaluating a type of a dry eye and a dynamic phase of a tear layer.

### Citation List

### Patent Literature

Patent Literature 1: JP 2018-47083 A
Patent Literature 2: WO 2018/203515 A

### Summary of Invention

### Technical Problem

In the technology of the related art, it is necessary to construct a system that automatically performs determination according to an artificially decided evaluation criterion at the time of evaluation, and since the decision of the evaluation criterion involves subjectivity, it is difficult to improve the reliability of the evaluation.

The present invention has been made in view of the above problems, and an object of the present invention is to provide a technology that enables objective classification.

### Solution to Problem

In order to achieve the aforementioned object, an examination target classification system includes an examination target image acquisition unit that acquires an examination target image which is an image of an examination portion of an anterior eye portion or a region around the anterior eye portion, a classification unit that inputs the examination target image to a machine learning model which classifies a state of the examination portion based on the image of the examination portion, and acquires a classification result, and a display unit that displays the classification result.

That is, in the examination target classification system, the machine learning model that classifies the state of the examination portion is generated in advance based on the image of an examination portion of the anterior eye portion or a region around the anterior eye portion. Accordingly, when the image of an examination portion of the anterior eye portion or a region around the anterior eye portion is acquired, the examination target can be automatically classified based on the machine learning model. The machine learning model is obtained by preparing teaching data in which that an image is associated with a classification result for learning and performing machine learning. Accordingly, according to the machine learning model, classification can be performed without depending on subjectivity.

### Brief Description of Drawings

FIG. 1 is a diagram schematically illustrating an optical system included in an examination target classification system.
FIG. 2 is a block diagram illustrating an internal configuration of a head unit and a main body unit.
FIG. 3A is a block diagram illustrating a configuration of a machine learning system, and FIG. 3B is a flowchart of machine learning processing.
FIG. 4 is a diagram illustrating a model of machine learning.
FIG. 5A is a diagram illustrating an example of a combination of light adjustment members, FIG. 5B is a diagram illustrating a captured image, and FIG. 5C is a diagram illustrating an examination target image.
FIG. 6A is a flowchart of examination target classification processing, and FIG. 6B is a diagram illustrating an example of a display form of a classification result.
FIGS. 7A and 7B are diagrams illustrating display examples of classification results.

### Description of Embodiments

Here, an embodiment of the present invention will be described in the following order.
(1) Configuration of ophthalmic apparatus:
(2) Generation of machine learning model:
(3) Examination target classification processing:
(4) Other embodiments:

### (1) Configuration of ophthalmic apparatus:

An examination target classification system according to an embodiment of the present invention is included in an ophthalmic apparatus. The ophthalmic apparatus may be configured in various forms, and for example, a configuration including a head unit including an optical system and a main body unit can be assumed. In this case, for example, the main body unit is installed at an installation site such as a desk, and the head unit is movable relative to the main body unit. A control unit that controls each unit of the ophthalmic apparatus and classifies a state of an examination portion is included in the main body unit. The head unit is movable relative to the ophthalmic apparatus, and alignment with respect to an eye to be examined is performed by the movement of the head unit. Of course, the form of the ophthalmic apparatus is an example, and various other forms may be used.

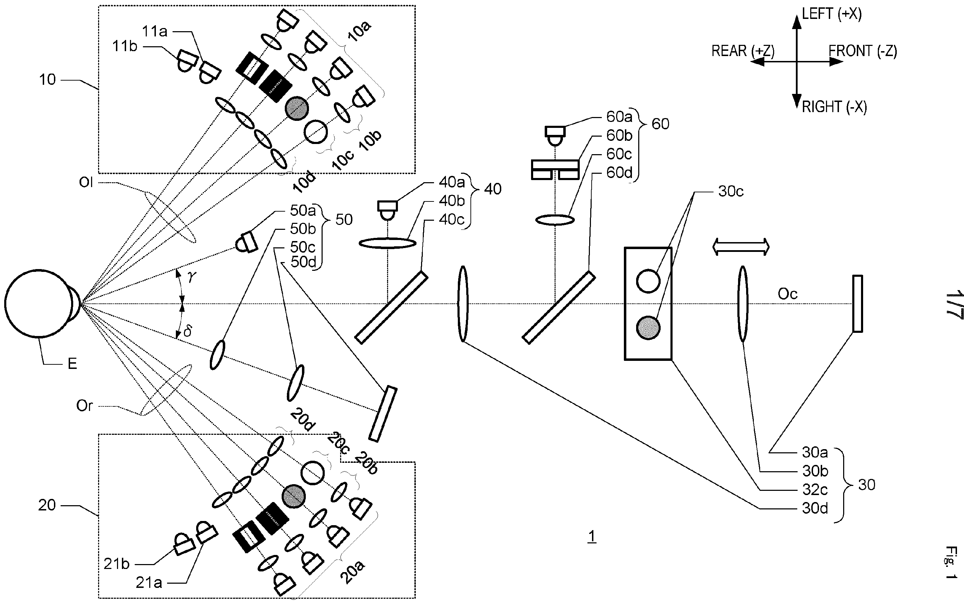

The optical system may be capable of capturing an examination portion of an examination target, and the arrangement and optical components thereof are not limited as long as the optical system can capture the examination portion. FIG. 1 is a diagram schematically illustrating an optical system of an ophthalmic apparatus 1 according to the present embodiment. In the present embodiment, as illustrated in an upper right of FIG. 1, upper, lower, left, right, front, and rear are expressed based on a direction seen from a subject having an eye to be examined E. In the drawing, a front side is an upper direction, and a back side is a lower direction. In the ophthalmic apparatus 1 according to the present embodiment, an X direction, a Y direction, and a Z direction are associated with these directions. In the present embodiment, a front-rear direction is a Z-axis direction, a rear side is a +Z direction, an upper-lower direction is a Y-axis direction, an upper direction is a +Y direction, a left-right direction is an X-axis direction, and a left side is a +X direction. Of course, correspondences between directions and axes and positive and negative directions may be different.

In the present embodiment, a plurality of examinations can be performed on the eye to be examined E, and as a part of the examinations, a function of classifying the examination target from an image of the examination portion of the eye to be examined E or the region around the eye to be examined E can be executed. As the classification, there is classification of degrees of severity of hyperemia of the eye to be examined E. The ophthalmic apparatus 1 has a function of capturing an image of the eye to be examined for various examinations. In the present embodiment, the ophthalmic apparatus performs capturing by light projected in a diagonal direction from a position other than the front of a subject S. In order to prevent the light in the diagonal direction from being disturbed by the nose of the subject S, the ophthalmic apparatus is configured such that the light is projected from a right side to a right eye of the eye to be examined and is projected from a left side to a left eye. That is, two projection optical systems are provided.

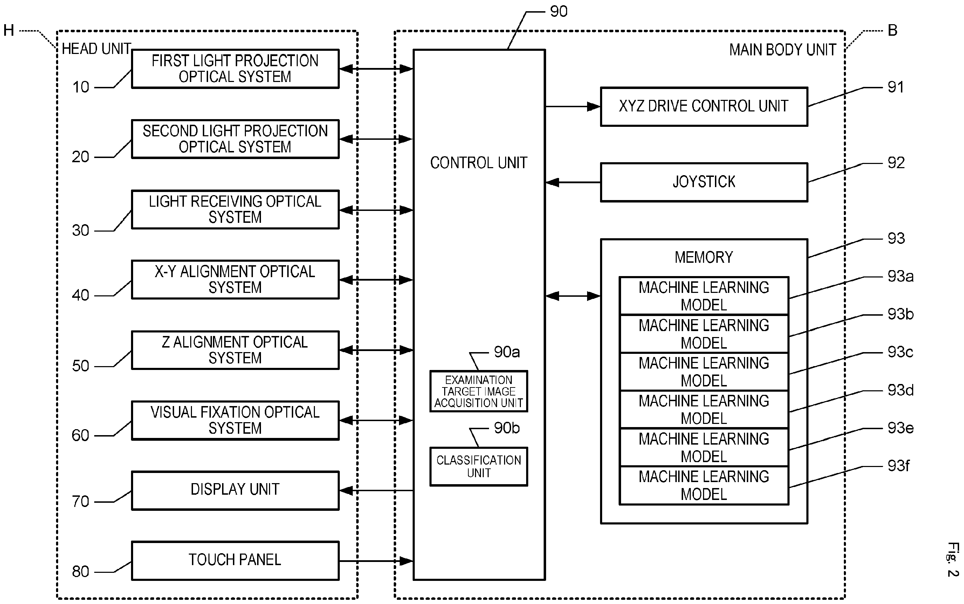

FIG. 2 is a block diagram illustrating a configuration of the ophthalmic apparatus 1 focusing on the control of the optical system or the like. As illustrated in FIG. 2, a main body unit B of the ophthalmic apparatus 1 includes an XYZ drive control unit 91 that moves a head unit H in XYZ directions, a joystick 92 that instructs adjustment of a spatial position of the head unit H or the like, and a memory 93 used in control processing of a control unit 90.

The head unit H includes a first light projection optical system 10 for projecting light to the left eye, a second light projection optical system 20 for projecting light to the right eye, a light receiving optical system 30 for performing capturing, an X-Y alignment optical system 40 for performing alignment on am X-Y direction, a Z alignment optical system 50 for performing alignment in the Z direction, a visual fixation optical system 60 for achieving a visual fixation state, a display unit 70 that displays the image of the captured eye to be examined, a user interface for selecting an examination mode, and the like, and a touch panel 80 that receives instructions such as examination items.

### (First light projection optical system)

In FIG. 1, the positions of the optical systems are illustrated such that a direction when the front side is viewed from the eye to be examined E and an optical axis Oc of the light receiving optical system 30 extending in the front-rear direction inside the head unit H coincide with each other. The first light projection optical system 10 is disposed on a left side with respect to the optical axis Oc, and includes a plurality of first light sources 10a, lenses 10b and 10d, and a light adjustment member 10c. As illustrated in FIG. 1, the lens 10b, the light adjustment member 10c, and the lens 10d are provided on each optical axis extending from each first light source 10a.

In the present embodiment, the first light source 10a is a white LED, and can output first measurement light including light having a wavelength over the entire range of visible light. The first light source 10a is oriented to a diagonal rear direction, and is configured such that an optical axis Ol passing through the lenses 10b and 10d intersects the optical axis Oc. The optical axis Ol is a general term for each optical axis between each of the first light sources 10a and the eye to be examined E.

The light adjustment member 10c is disposed between the lenses 10b and 10d. The light adjustment member 10c is a member for adjusting the light output from the first light source 10a. In the present embodiment, the light adjustment member 10c is a diffuser, a wavelength selection filter, and a slit light generation member. The diffuser is a filter that diffuses the light output from the first light source 10a, and is a light adjustment member that prevents the first measurement light having a spot shape from being projected onto the eye to be examined E and prevents reflected light reflected from the eye to be examined E from becoming locally strong.

In the present embodiment, the wavelength selection filter is a blue band-pass filter. That is, the wavelength selection filter is a light adjustment member that attenuates light other than blue from the first measurement light output from the first light source 10a and transmits blue light. The slit light generation member is a light adjustment member having a slit. The slit light generation member is disposed on an optical axis of the first light source 10a, and thus, the light source generation member causes a part of the first measurement light output from the first light source 10a to pass through, and converts the light into slit light of which a shape of a cross section in a direction perpendicular to the optical axis is a substantially rectangular shape. The slit light generation member of the present embodiment includes two types of members having different slit widths.

As described above, the light adjustment member 10c is constituted by a plurality of light adjustment members. In the present embodiment, any one of the first light sources 10a is turned on, and thus, the light output from each first light source 10a passes through the optical axis and reaches the eye to be examined E. In the present embodiment, the optical axis through which light passes as stated above is referred to as an effective optical axis. In the present embodiment, any one of the first light sources 10a is turned on. In this case, the light adjustment member 10c present between the light source that is turned on and the eye to be examined E is in a state of being disposed on the effective optical axis. The light adjustment member 10c present between the light source that is not turned on and the eye to be examined E is not disposed on the effective optical axis.

In the present embodiment, the first light projection optical system 10 includes light sources 11a and 11b other than the first light sources 10a. The light output from these light sources 11a and 11b is projected onto the eye to be examined E and illuminates the eye to be examined E. In the present embodiment, since the light output from the light sources 11a and 11b is not adjusted, the light output from the light sources 11a and 11b is projected onto the eye to be examined E without passing through the light adjustment member 10c. Accordingly, in the present embodiment, the light sources 11a and 11b are arranged so as to be closer to the eye to be examined E than the light adjustment member 10c. When the adjustment using the light adjustment member is necessary, the light sources 11a and 11b may be arranged so as to be closer to the rear side than the light adjustment member 10c when viewed from the eye to be examined E, and thus, the adjustment using the light adjustment member may be performed. The types of the light sources 11a and 11b are not limited, and, for example, LEDs and the like that output infrared rays are used.

### (second light projection optical system)

The second light projection optical system 20 is disposed on a right side with respect to the optical axis Oc, and includes a second light source 20a, lenses 20b and 20d, and a light adjustment member 20c. The elements constituting the second light projection optical system 20 and the configurations of the light sources 21a and 21b are the same as those of the first light projection optical system 10. In the present embodiment, the first light projection optical system 10 and the second light projection optical system 20 are arranged on opposite sides with the optical axis Oc interposed therebetween. In the present embodiment, the optical axes Ol, Oc, and Or are present on the same plane (on a horizontal plane), and the optical axis Ol and the optical axis Or are symmetrical with respect to the optical axis Oc. The members of the first light projection optical system 10 and the second light projection optical system 20 are arranged at positions line-symmetrical with respect to the optical axis Oc. Although the light adjustment member 10c and the light adjustment member 20c are the same members in the present embodiment, the arrangement of the light adjustment members may not be line-symmetrical.

### (Light receiving optical system)

The light receiving optical system 30 includes an image sensor 30a, lenses 30b and 30d, and a light receiving adjustment unit 32c on the optical axis Oc extending in the front-rear direction. The optical axis Oc is a linear axis that passes through centers of the image sensor 30a and the lenses 30b and 30d, and is assumed to pass through a corneal apex when the eye to be examined E is aligned at a predetermined position.

When the first measurement light is projected onto the eye to be examined E by the first light projection optical system 10 or when the second measurement light is projected onto the eye to be examined E by the second light projection optical system 20, the projected light is reflected by the eye to be examined E. The reflected light travels along the optical axis Oc, passes through the lens 30d, the light receiving adjustment unit 32c, and the lens 30b, and is received by the image sensor 30a. In the present embodiment, the image sensor 30a is an image sensor that detects the intensity of each color of RGB (R: red, G: green, B: blue) for each pixel, and generates a color image.

The light receiving adjustment unit 32c is disposed between the lenses 30b and 30d. In the present embodiment, the light receiving adjustment unit 32c includes a plurality of wavelength selection filters as light adjustment members 30c. In the present embodiment, the wavelength selection filters are a green band-pass filter and a long-pass filter. The green band-pass filter is a light adjustment member that attenuates light other than green from the reflected light reflected by the eye to be examined E and transmits the green light. The long-pass filter is a light adjustment member that attenuates light on a short wavelength side from the reflected light reflected by the eye to be examined E and transmits light on a long wavelength side. A wavelength range to be attenuated or transmitted in the long-pass filter may be various wavelength ranges, and for example, a filter that transmits the red light, a filter that transmits infrared rays, or the like may be used.

As described above, the light receiving adjustment unit 32c includes the plurality of light adjustment members 30c, and includes a mechanism that switches between a state in which any one of the light adjustment members is disposed on the optical axis Oc and a state in which the light adjustment member is not disposed on the optical axis Oc (state in which light passes through a portion at which the light adjustment member is not present). Accordingly, the reflected light reflected from the eye to be examined E is received, as green light, light obtained by attenuating the light on the short wavelength side, or unadjusted reflected light, by the image sensor 30a.

When the light is projected onto the eye to be examined E, since the reflected light from the eye to be examined E passes through the optical axis Oc, the optical axis Oc is in an effective state. Thus, any one of the wavelength selection filters is disposed on the optical axis Oc by the light receiving adjustment unit 32c, and thus, the light adjustment member 30c is in a state of being disposed on the effective optical axis. In the light receiving adjustment unit 32c, the light adjustment member 30c may be selectable by various mechanisms. For example, it is possible to adopt a configuration in which the light adjustment member 30c is moved by a driving force of the motor and the member disposed on the optical axis Oc is selectable.

As described above, in the present embodiment, the light adjustment member 10c and the light adjustment member 20c are the same. That is, each of the first light projection optical system 10 and the second light projection optical system 20 includes the same diffuser, the same blue band-pass filter, and the same slit light generation member. On the other hand, the light adjustment member 30c is different from the light adjustment member 10c and the light adjustment member 20c. That is, the light receiving adjustment unit 32c includes the green band-pass filter and the long-pass filter. These filters are not provided in either the light adjustment member 10c or the light adjustment member 20c.

As described above, in the present embodiment, the light adjustment members 10c and 20c for adjusting the projected light and the light adjustment member 30c for adjusting the light reflected by the eye to be examined E are different from each other. Thus, the same adjustment is not performed on the projected light and the reflected light, and different adjustments can be performed. Accordingly, the light adjustment members can be efficiently arranged in the head unit H. Each of the light adjustment member 10c, the light adjustment member 20c, and the light adjustment member 30c includes the plurality of light adjustment members. Accordingly, the light adjustment member is selected, and thus, various adjustments can be performed on the projected light and the reflected light.

In FIG. 1, the lens 30b is schematically represented. The lens 30b in the present embodiment is an autofocus mechanism (not illustrated) that achieves an autofocus function. That is, the lens 30b includes one or more lenses movable in an optical axis Oc direction, and the control unit 90 can change a position of the optical axis Oc direction at which an image of the eye to be examined E is formed by outputting a control signal to the light receiving optical system 30. In the present embodiment, autofocus is achieved by capturing a plurality of images obtained by changing the position of the lens 30b and acquiring the image having the best image quality. The image quality may be evaluated such that as the image quality becomes better, a focusing state becomes better. For example, it is possible to adopt a configuration in which as contrast and brightness become larger, the image quality is evaluated as becoming better.

When capturing is started, the control unit 90 illustrated in FIG. 2 controls the autofocus mechanism including the lens 30b of the light receiving optical system 30, and performs capturing using the image sensor 30a in a predetermined cycle while moving the lens 30b from a predetermined movement start position to a movement end position. The control unit 90 performs control such that focusing in a predetermined region (for example, a rectangular region) including the examination portion of the eye to be examined E is performed. In the present embodiment, in order to determine the focusing state based on the image quality, the control unit 90 compares the image qualities in the regions including the examination portion between the images, acquires the image having the best image quality, and records the acquired image in the memory 93. As a result, it is possible to record the image focused on the examination portion.

Of course, various methods may be adopted for the autofocus method. FIG. 1 illustrates that autofocus is executable by adding an arrow to a portion above the lens 30b. As described above, according to the configuration in which the autofocus is executable, it is possible to automatically capture a clear image, and the examination becomes easy. An examiner can use the image on which the examination portion is clearly captured without being aware that focusing is to be performed on different positions in the image depending on a difference between examination portions.

With the above configuration, in the light receiving optical system 30, the eye to be examined E can be captured based on the reflected light obtained by reflecting the light projected by the first light projection optical system 10 and the second light projection optical system 20 (or the other optical system to be described later) from the eye to be examined E. The captured image is recorded in the memory 93.

### (X-Y alignment optical system)

The X-Y alignment optical system 40 includes an X-Y alignment light source 40a, a lens 40b, and a half mirror 40c. In the X-Y alignment optical system 40, the X-Y alignment light source 40a, the lens 40b, and the half mirror 40c are arranged in this order from a far side in a direction perpendicular to the optical axis Oc. The X-Y alignment light source 40a is oriented such that an optical axis thereof is perpendicular to the optical axis Oc.

The half mirror 40c is oriented such that output light of the X-Y alignment light source 40a passed through the lens 40b is reflected in the optical axis Oc direction. Accordingly, the output light output from the X-Y alignment light source 40a passes through the lens 40b, is reflected by the half mirror 40c, reaches the eye to be examined E, and is reflected. The reflected light reflected by the eye to be examined E travels along the optical axis Oc, passes through the half mirror 40c, passes through the lens 30d, a half mirror 60d, the light receiving adjustment unit 32c, and the lens 30b, and is received by the image sensor 30a.

In the present embodiment, when the corneal apex of the eye to be examined E is present on the optical axis Oc in the X-Y plane (in the plane in the upper, lower, left, and right directions), the X-Y alignment optical system 40 is designed such that the output light of the X-Y alignment light source 40a is present at a reference position (for example, center) of the image sensor 30a. Accordingly, when a relationship between a position of a bright spot detected by the image sensor 30a and the reference position is specified, the direction and the moving amount in the X-Y plane in which the head unit H is to be moved such that the corneal apex of the eye to be examined E is present on the optical axis Oc can be specified.

Thus, when the X-Y alignment is performed, the control unit 90 controls the X-Y alignment optical system 40 to turn on the X-Y alignment light source 40a. In this state, the control unit 90 detects alignment light output by the X-Y alignment light source 40a by the image sensor 30a included in the light receiving optical system 30. The control unit 90 specifies the moving direction and the moving amount of the head unit H while referring to the detection result of the alignment light. The XYZ drive control unit 91 includes a mechanism for moving the head unit H together with the optical system in the XYZ directions. Thus, the control unit 90 instructs the moving direction and the moving amount of the head unit H to the XYZ drive control unit 91. As a result, the head unit H and an internal optical system thereof move as one, and are aligned in the X-Y direction with respect to the eye to be examined E.

### (Z alignment optical system)

The Z alignment optical system 50 includes a Z alignment light source 50a, lenses 50b and 50c, and a position detection sensor 50d. In the Z alignment optical system 50, the Z alignment light source 50a is attached to the head unit H such that the optical axis is oriented in a direction of an angle γ in the horizontal plane with respect to the optical axis Oc. In the Z alignment optical system 50, the lenses 50b and 50c and the position detection sensor 50d are arranged in order from the eye to be examined E side along an optical axis inclined in a direction of an angle δ in the horizontal plane with respect to the optical axis Oc.

In the present embodiment, the angle γ and the angle δ are the same, and when the corneal apex of the eye to be examined E is on the optical axis Oc and is presented at a predetermined position in the Z direction, the Z alignment optical system 50 is designed such that output light of the Z alignment light source 50a is present at a reference position (for example, center) of the position detection sensor 50d. Accordingly, when a relationship between a position of a bright spot detected by the position detection sensor 50d and the reference position is specified, the moving direction and the moving amount on a Z axis in which the head unit H is to be moved such that the corneal apex of the eye to be examined E is on the optical axis Oc and is present at the predetermined position can be specified.

Thus, when Z alignment is performed, the control unit 90 controls the Z alignment optical system 50 to turn on the Z alignment light source 50a. In this state, the control unit 90 detects alignment light output by the Z alignment light source 50a by the position detection sensor 50d. The control unit 90 specifies the moving direction and the moving amount of the head unit H while referring to the detection result of the alignment light. The XYZ drive control unit 91 includes a mechanism for integrally moving the head unit H with an internal optical system or the like in the XYZ directions. Thus, the control unit 90 instructs the moving direction and the moving amount of the head unit H to the XYZ drive control unit 91. As a result, the head unit H and the internal optical system move integrally, and are aligned in the Z direction with respect to the eye to be examined E.

### (Visual fixation optical system 60)

The visual fixation optical system 60 includes a visual fixation light source 60a, a visual fixation target 60b, a lens 60c, and the half mirror 60d. In the visual fixation optical system 60, the visual fixation light source 60a, the visual fixation target 60b, the lens 60c, and the half mirror 60d are arranged in this order from a far side in the direction perpendicular to the optical axis Oc. The visual fixation light source 60a is oriented such that an optical axis thereof is perpendicular to the optical axis Oc. The half mirror 60d is oriented such that output light of the visual fixation light source 60a passed through the lens 60c and the visual fixation target 60b is reflected in the optical axis Oc direction.

The visual fixation target 60b includes a pinhole and a diffuser (the diffuser may be omitted). In the above configuration, the output light output from the visual fixation light source 60a passes through the diffuser and pinhole of the visual fixation target 60b, passes through the lens 60c, is reflected by the half mirror 60d, and reaches the eye to be examined E. Thus, an image is formed on the retina of the eye to be examined E. Thus, the pinhole of the visual fixation target 60b is conjugate with a position of the fundus of the eye to be examined E. In the optical system illustrated in FIG. 1, the arrangement of the lenses and the like is a schematic diagram, and may differ from an actual arrangement. The eye to be examined E is in the visual fixation state by fixing the light passed through the visual fixation target 60b.

### (2) Generation of machine learning model:

In the ophthalmic apparatus 1 according to the present embodiment, the eye to be examined E can be captured, and the examination portion included in the captured image can be classified. In the present embodiment, the classification is executed based on a machine learning model. Thus, in the present embodiment, a plurality of machine learning models for classifying the state of the examination portion based on the image of the examination portion is generated by machine learning and is recorded in the memory 93 of the ophthalmic apparatus 1 (machine learning models 93a to 93f).

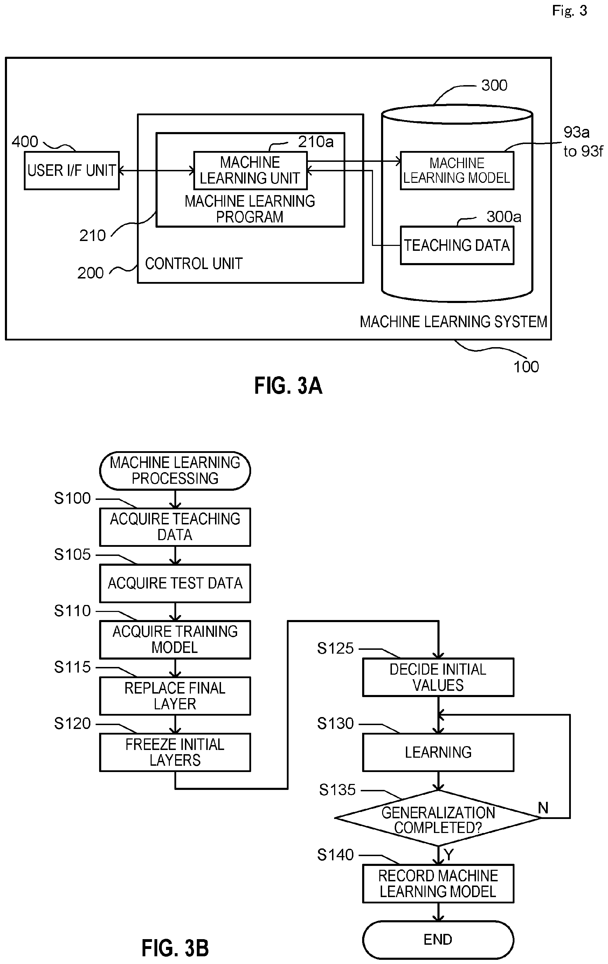

FIG. 3A is a block diagram illustrating a hardware configuration of a machine learning system 100 that generates the machine learning models 93a to 93f, and FIG. 3B is a flowchart illustrating machine learning processing. The machine learning system 100 includes a control unit 200, a recording medium 300, and a user I/F unit 400.

The control unit 200 includes a CPU, a RAM, a ROM, and a GPU (all not illustrated), and can execute various programs stored in the recording medium 300 or the like. The user I/F unit 400 includes a display unit such as a display and an input unit such as a keyboard or a mouse. The control unit 200, the recording medium 300, and the user I/F unit 400 may be an integrated computer, or may have a configuration in which at least a part thereof is another apparatus and is connected by a USB cable or the like.

In the present embodiment, the control unit 200 can execute a machine learning program 210. The machine learning program 210 is a program that causes the control unit 200 to execute a function of performing machine learning and acquiring a model that outputs, as an examination result, a state of an examination portion included in an examination target image captured by the ophthalmic apparatus 1. When the machine learning program 210 is executed, the control unit 200 functions as a machine learning module 210a.

The machine learning processing is executed in a state in which teaching data 300a is prepared in advance. The control unit 200 optimizes a model of a neural network based on the teaching data 300a, and stores, as the machine learning models 93a to 93f, the optimized model in the recording medium 300.

Machine learning may be performed by various methods, but here, an example in which a model including a convolutional neural network (CNN) is optimized by machine learning will be described. In order to optimize the model by machine learning, the teaching data 300a is defined by associating an image to be input to the model with information indicating the classification result of the examination portion included in the image. In the present embodiment, the classification corresponds to the degree of severity of the symptom of the examination portion. For example, when degrees of severity of hyperemia in (a white eye portion of) an anterior eye portion are examination targets, the degrees of severity of the hyperemia are classified into a plurality of groups.

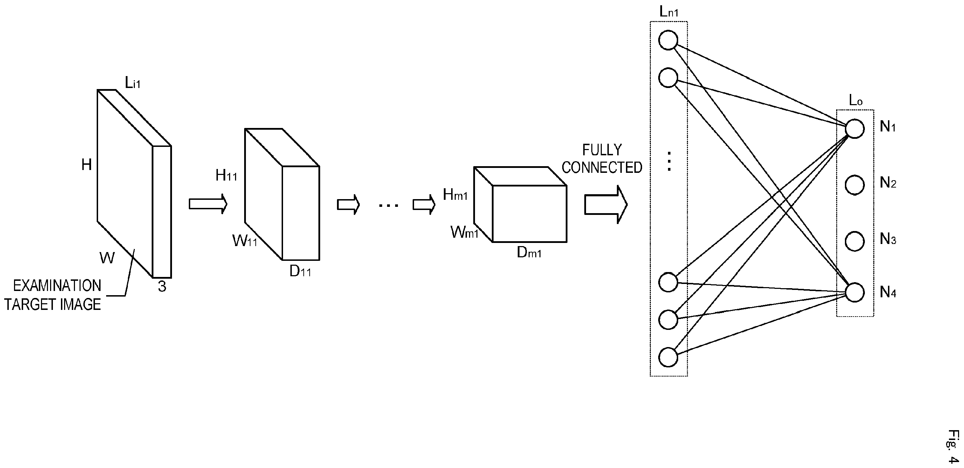

FIG. 4 is a diagram schematically illustrating a structure of the model used in the present embodiment. In FIG. 4, a change in a data format of the CNN is illustrated by a change in a cuboid, and nodes of the neural network are illustrated by white circles. In the model of the present embodiment, the examination target image is used as input data to an input layer Lᵢ₁ of the CNN, and an estimated value is output to an output layer Lo that outputs the classification result via a fully connected layer Lₙ₁. The examination target image input to the CNN is a vertical H pixel and a horizontal W pixel, and colors of the pixels are expressed by gradation values of three channels of R: red, G: green, and B: blue.

Accordingly, in FIG. 4, the image of the input layer Lᵢ₁ is schematically illustrated as a cuboid having vertical H, horizontal W, and depth 3. FIG. 4 illustrates an example in which after the image is input to the input layer, the image is converted into (H₁₁ × W₁₁ × D₁₁) output values via the CNN, that is, through a convolution operation by a predetermined number of filters having a predetermined size, an operation using an activation function, and an operation of a pooling layer. FIG. 4 illustrates an example in which the image is subsequently converted into (Hₘ₁ × Wₘ₁ × Dₘ₁) output values through a plurality of layers (not illustrated). After the (Hₘ₁ × Wₘ₁ × Dₘ₁) output values are obtained by the CNN, output values of nodes N₁ to N₄ corresponding to the plurality of groups are obtained by the output layer Lo through the fully connected layer Lₙ₁.

The output values of the nodes N₁ to N₄ indicate probabilities that the examination portion of the input image belongs to each of the plurality of groups. For example, the groups are called groups 1, 2, 3, and 4 in ascending order of the degree of severity, and each of the groups 1, 2, 3, and 4 is determined to be associated with each of the nodes N₁ to N₄ in advance. When the outputs of the nodes N₁ to N₄ are 0.95, 0.03, 0.01, and 0.01, the probabilities that the state of the examination portion belongs the groups 1, 2, 3, and 4 are estimated as 95%, 3%, 1%, and 1%. In the present embodiment, the sum of the output values of the nodes N₁ to N₄ is 1.

The teaching data 300a is data in which an image in a format that can be input to such a model is associated with the output values of the nodes N₁ to N₄. In the teaching data 300a, the output values of the nodes N₁ to N₄ are 1 for any node and 0 for the other nodes. The format that can be input to the model is a three-color image having an H pixel in a vertical direction and a W pixel in a horizontal direction, but a portion other than the examination portion is further removed from the image including the examination portion in the present embodiment.

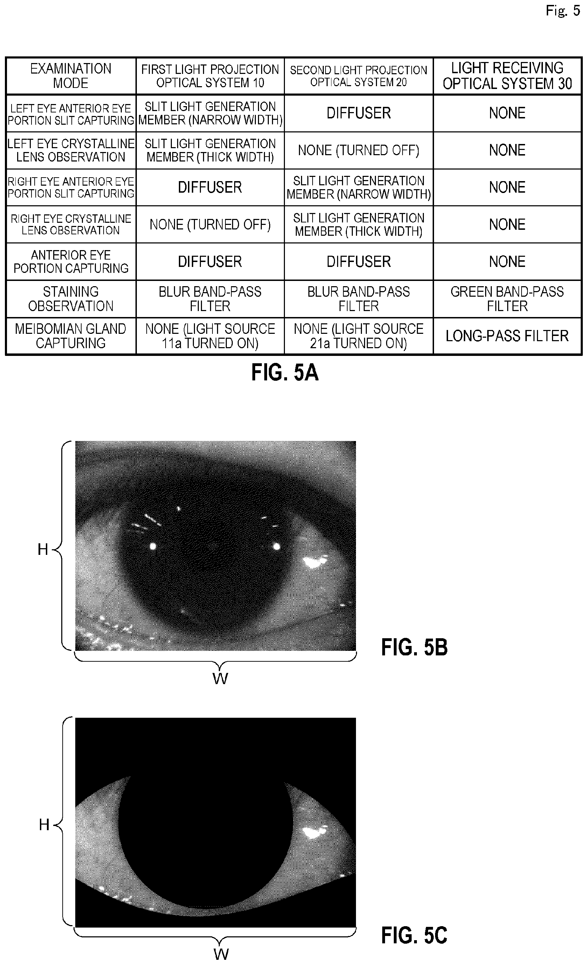

FIG. 5B illustrates an example of an image of the anterior eye portion for examining the degree of severity of the hyperemia. Since the hyperemia occurs in the white eye portion of the anterior eye portion, a portion other than the white eye portion is not necessary to classify the degree of severity of the hyperemia. Thus, in this case, an image in which the white eye portion is left and the other portion is removed (an image in which the other portion is replaced with a special color (for example, black)) as illustrated in FIG. 5C is used as the teaching data 300a.

When the machine learning processing is started, the control unit 200 acquires the teaching data 300a by the function of the machine learning module 210a (step S100). Subsequently, the control unit 200 acquires test data by the function of the machine learning module 210a (step S105). In the present embodiment, the control unit 200 extracts a part of the teaching data 300a and uses this part as the test data for confirming whether or not the generalization of the learning is performed. The test data is not used for machine learning.

Subsequently, the control unit 200 acquires training models by the function of the machine learning module 210a (step S110). In the present embodiment, the control unit 200 acquires a parameter (a filter, an activation function, or the like) for each node for each layer indicating the training model. In the present embodiment, the training model is a model in which machine learning is performed in advance based on teaching data different from the teaching data 300a. For example, the training model is a model in which machine learning is performed based on teaching data in which an output different from the degree of severity of the hyperemia is associated with an image different from the examination target image.

Such a training model can be obtained by using the Internet or the like, but since machine learning is performed based on teaching data different from the teaching data 300a in which the examination target image is associated with the classification result, such a training model is different from the model illustrated in FIG. 4.

Thus, the control unit 200 replaces the final layer by the function of the machine learning module 210a (step S115). That is, the control unit 200 replaces the output layer of the training model acquired in step S110 with the nodes N₁ to N₄ illustrated in FIG. 4, and connects the nodes N₁ to N₄ and the immediately preceding layer by full connection. The connection is achieved by redefining the filter, the activation function, and the like. Of course, when the format of the input layer is different from the format of the examination target image, the input layer may be replaced. The format of the examination target image may be defined according to the format of the input layer of the training model.

Subsequently, the control unit 200 freezes initial layers by the function of the machine learning module 210a (step S120). That is, in order to perform efficient learning, the control unit 200 uses the initial layers (for example, 10 layers in the training model having 100 layers or more) as layers in which parameters are not updated by learning. As a result, machine learning can be converged at an early stage. Of course, the number of layers to be frozen is not limited, and an intermediate layer and a layer near an output side may be frozen. When machine learning is completed, freezing may not be performed.

Subsequently, the control unit 200 decides an initial value by the function of the machine learning module 210a (step S125). That is, the control unit 200 decides, as an initial value of a learning target, a parameter of the training model acquired in step S110 for a layer other than the layers frozen in step S120. The final layer has a predetermined initial value. Of course, the initial value may be adjusted such that the parameter is optimized in a learning procedure.

Subsequently, the control unit 200 performs learning by the function of the machine learning module 210a (step S130). That is, the control unit 200 inputs the image of the teaching data 300a acquired in step S100 into the model after the replacement is performed in step S115, and acquires an output value. The control unit 200 specifies an error based on a loss function indicating a difference between the output value and the classification result associated with the teaching data 300a. The control unit 200 updates the parameter by a predetermined optimization algorithm, for example, a stochastic gradient descent method or the like. That is, the control unit 200 repeats processing of updating the parameter a predetermined number of times based on differentiation of the loss function by the parameter such that the error becomes small. The parameter to be updated here is a parameter that is not frozen in step S120.

By doing this, when the parameter is updated a predetermined number of times, the control unit 200 determines whether or not the generalization of the model is completed (step S135). That is, the control unit 200 inputs the test data acquired in step S105 into the model and acquires the output value indicating the classification result of the image. The control unit 200 acquires a number in which the output value and the classification result associated with the test data coincide with each other, and acquires estimation accuracy by dividing the acquired number by the number of samples. In the present embodiment, the control unit 200 determines that the generalization is completed when the estimation accuracy is equal to or higher than a threshold value.

In addition to the evaluation of generalization performance, the validity of a hyperparameter may be verified. That is, in a configuration in which a hyperparameter that is a variable quantity other than a variable parameter to be learned, for example, the number of nodes or the like is tuned, the control unit 200 may verify the validity of the hyperparameter based on verification data. The verification data may be acquired by extracting the verification data from the teaching data 300a in advance by the same processing as in step S110 and securing the verification data as data not used for training.

When it is not determined in step S135 that the generalization of the model is completed, the control unit 200 repeats step S130. That is, the processing of updating the variable parameter to be learned is further performed. On the other hand, when it is determined in step S135 that the generalization of the model is completed, the control unit 200 records the machine learning model (step S140). That is, the control unit 200 records, as the machine learning model, the model for which the generalization is completed in the recording medium 300.

In the present embodiment, the plurality of machine learning models is prepared in advance. Thus, at least S100 to S140 of the machine learning processing are performed as many times as the number of machine learning models, and the obtained machine learning models are recorded in the recording medium 300. In the present embodiment, six models are acquired by machine learning, and these models are represented as the machine learning models 93a to 93f in FIG. 3A. The machine learning models 93a to 93f have different training models on which these machine learning models are based. That is, in the present embodiment, since the machine learning models 93a to 93f are acquired by modifying a part of the layers of the machine-learned model, these machine learning models 93a to 93f partially retain the features of the training models on which these machine learning models are based. Thus, even though classification accuracy for an examination target image having a specific feature is lowered by some models, when the plurality of machine learning models 93a to 93f is used, there is a possibility of being compensated by other models. Accordingly, according to the present embodiment, it is possible to increase the possibility that the classification is performed accurately.

By doing this, when the machine learning models 93a to 93f are obtained, the machine learning models 93a to 93f are recorded in the memory 93 of the ophthalmic apparatus 1 by any transfer method. The ophthalmic apparatus 1 according to the present embodiment can execute the examination of the examination portion in a state in which the machine learning models 93a to 93f are recorded in the memory 93. In the present embodiment, six machine learning models 93a to 93f are used to classify one type of examination portion. Accordingly, when N types of examination portions are to be classified, 6N machine learning models are generated in advance and are recorded in the memory 93.

### (3) Examination target classification processing:

Hereinafter, examination target classification processing of capturing the eye to be examined E by the ophthalmic apparatus 1 and classifying the examination portion included in the captured image will be described. The control unit 90 functions as an examination target image acquisition module 90a and a classification module 90b by executing the examination target classification program. The examination target image acquisition module 90a is a function of performing control such that the first light source 10a and the second light source 20a are turned on or off and control such that the examination target image that is an image of the examination portion is acquired. The classification module 90b is a function of inputting the examination target image into the machine learning model and acquiring the classification result. The examination target classification processing is executed by the control unit 90 functioning as the examination target image acquisition module 90a and the classification module 90b.

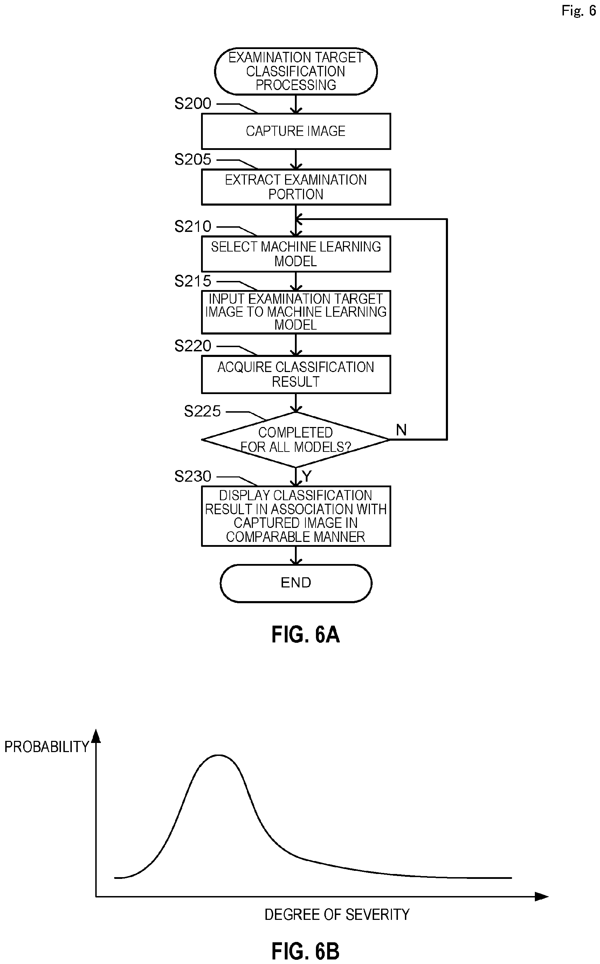

FIG. 6A is a flowchart illustrating the examination target classification processing. In the ophthalmic apparatus 1 according to the present embodiment, the examination portions as the examination targets are captured, and the examination portions are classified into the plurality of groups based on the capturing result. In the present embodiment, the ophthalmic apparatus 1 has a plurality of examination modes having different examination targets, and the control unit 90 receives the selection of the examination mode by the examiner via the touch panel 80 by the function of the examination target image acquisition module 90a.

The control unit 90 receives an instruction to start the examination target classification processing by the examiner via the touch panel 80 by the function of the examination target image acquisition module 90a. When the start instruction is received, the control unit 90 executes the examination target classification processing corresponding to the examination portion for each examination mode. When the examination target classification processing is started, the control unit 90 captures the image by the function of the examination target image acquisition module 90a (step S200). At this time, the subject sets his or her head at a predetermined position of the head unit H.

The control unit 90 indicates the light sources to be turned on for the first light projection optical system 10 and the second light projection optical system 20 according to the examination mode. In each of the first light projection optical system 10 and the second light projection optical system 20, the instructed light source is turned on, and the uninstructed light source is not turned on. As a result, the light adjustment member present on the optical axis of the light source that is turned on is in a state of being selected. The control unit 90 instructs the light adjustment member 30c to be used in the light receiving adjustment unit 32c according to the examination mode by the function of the examination target image acquisition module 90a. In the light receiving optical system 30, the light receiving adjustment unit 32c drives a mechanism (not illustrated), and moves the instructed light adjustment member 30c on the optical axis Oc. As a result, in each of the first light projection optical system 10, the second light projection optical system 20, and the light receiving optical system 30, the light adjustment members corresponding to the examination modes illustrated in FIG. 5A are arranged on the optical axis.

FIG. 5A is a diagram illustrating an example of a combination of the light adjustment members for each examination mode. In the present embodiment, the examination modes include anterior eye portion slit capturing, crystalline lens observation, anterior eye portion capturing, staining observation, and meibomian gland capturing. For example, when the anterior eye portion capturing is selected, the control unit 90 turns on the first light source 10a and the second light source 20a corresponding to the diffuser in the first light projection optical system 10 and the second light projection optical system 20. The control unit 90 operates the light receiving adjustment unit 32c in the light receiving optical system 30, and selects a state in which the light adjustment member 30c is not present on the optical axis. As a result, the anterior eye portion of the left eye or the right eye can be captured. As described above, the control unit 90 arranges the light adjustment members illustrated in FIG. 5A on the optical axis according to the examination portion for each examination mode.

In the examination mode illustrated in FIG. 5A, the anterior eye portion slit capturing is an examination mode in which the eye to be examined E is irradiated with thin slit light passed through a thin slit and an image in a state in which the cornea of the eye to be examined E or the like is illuminated linearly is captured. The crystalline lens observation is an examination mode in which the eye to be examined is irradiated with thick slit light passed through a thick slit and an image for observing the crystalline lens is captured.

The anterior eye portion capturing is an examination mode in which an image in a state in which the eye to be examined is illuminated with white light is captured. The staining observation is an examination mode in which an image of a state in which the eye to be examined E is stained with a fluorescein staining solution is captured. The meibomian gland capturing is a mode in which infrared rays are projected onto the eye to be examined E and meibomian glands present behind an upper eyelid or a lower eyelid of the eye to be examined E are captured. Accordingly, the first light source 10a of the first light projection optical system 10 and the second light source 20a of the second light projection optical system 20 are turned off, but the light sources 11a and 21a that output infrared rays are turned on. As described above, although different light adjustment members are used in combination according to the examination mode in the present embodiment, according to the present embodiment, the examiner can set to correctly combine the light adjustment members when the examination mode is instructed without being aware of the combination of the different light adjustment members for each examination mode.

In the present embodiment, the first light projection optical system 10 is used when the light is projected with the left eye of the subject S as the eye to be examined E, and the second light projection optical system 20 is used when the light is projected with the right eye of the subject S as the eye to be examined E. Thus, the light adjustment members in each light projection optical system may be arranged on the optical axis at least when the light is projected.

Accordingly, a configuration in which the light adjustment member is selected when the light source of each light projection optical system is turned on or the like can be adopted in addition to the configuration in which the light adjustment member is selected according to the selection of the examination mode. For example, the light source to be turned on in the anterior eye portion capturing may be either one (first light source 10a in the case of the left eye or second light source 20a in the case of the right eye) .

As described above, although the plurality of examination targets can be examined in the ophthalmic apparatus 1, an example in which the white eye portion of the anterior eye portion is the examination portion and the degree of severity of the hyperemia in the examination portion is classified will be mainly described.

When the light adjustment member is set, the control unit 90 controls the X-Y alignment optical system 40 and the Z alignment optical system 50 to execute the X-Y alignment and the Z alignment. The control unit 90 acquires an image in a state of being focused on the examination portion corresponding to the examination mode by using the autofocus mechanism, and records the acquired image in the memory 93. As a result, the captured image of the anterior eye portion illustrated in FIG. 5B is obtained.

When the image is captured, the control unit 90 then extracts the examination portion (step S205). That is, the examination portion is different for each examination mode, and the control unit 90 executes image processing of extracting the examination portion corresponding to the examination mode from the image captured in step S200. When the degree of severity of the hyperemia is examined, the control unit 90 extracts the white eye portion from the image captured in step S200 and removes the other portions (replaces the image data with a special color (for example, black)).

The extraction of the white eye portion may be executed by various methods, and the control unit 90 performs, for example, edge detection processing on the examination target image on which the anterior eye portion is captured, and specifies a lower end of the upper eyelid and an upper end of the lower eyelid. A portion which is not a black eye sandwiched between the lower end of the upper eyelid and the upper end of the lower eyelid is extracted as the examination portion. The obtained image is recorded, as the examination target image, in the memory 93. Of course, the extraction processing is an example, and the examination portion may be extracted based on the color of the image. FIG. 5C illustrates an example of the examination target image obtained by extracting the white eye portion as the examination portion from the image illustrated in FIG. 5B. According to the above configuration, it is possible to more accurately classify the state of the examination portion than when the image other than the examination portion is included.

The examination target image obtained by the extraction of the examination portion is in a format corresponding to the machine learning model. That is, the format of the input image (the number of vertical and horizontal pixels and the number of colors) for the machine learning models 93a to 93f is determined in advance. For example, each color of RGB is needed since a color image is needed to examine the degree of severity of the hyperemia. Since the size of the image that can be input to the machine learning models 93a to 93f is determined in advance, the examination target image is acquired such that the image of each color has a predetermined number of vertical pixels and horizontal pixels.

Subsequently, the control unit 90 classifies the state of the examination portion by executing steps S210 to S225. Specifically, the control unit 90 selects the machine learning models by the function of the classification module 90b (step S210). That is, the control unit 90 selects the machine learning models for examining the examination portion corresponding to the examination mode. One model that is not used in the processing of steps S210 to S225 is selected from among the selected machine learning models. For example, when the machine learning models 93a to 93f are models prepared for examining the degree of severity of the hyperemia, the control unit 90 selects one model that is not used in the processing of steps S210 to S225 from among these models.

Subsequently, the control unit 90 inputs the examination target image into the machine learning model by the function of the classification module 90b (step S215). That is, the control unit 90 inputs the examination target image acquired in step S205 to the machine learning model selected in step S210. That is, the control unit 90 performs an operation on each layer based on the machine learning model by using the examination target image as the input, and acquires the output values of the nodes N₁ to N₄ of the output layer.

Subsequently, the control unit 90 acquires the classification result by the function of the classification module 90b (step S220). That is, the control unit 90 acquires the output values of the nodes N₁ to N₄ acquired in step S215 as the probabilities that the degree of severity of the hyperemia of the white eye portion included in the examination target image belongs to the groups corresponding to the nodes N₁ to N₄. For example, when the outputs of the nodes N₁ to N₄ are 0.95, 0.03, 0.01, and 0.01, the control unit 90 estimates that the probabilities that the state of the examination portion belongs the groups 1, 2, 3, and 4 are 95%, 3%, 1%, and 1%.

Subsequently, the control unit 90 determines whether or not the processing is completed for all the models by the function of the classification module 90b (step S225). That is, the control unit 90 determines whether or not the processing of steps S210 to S225 is performed on all the machine learning models prepared for examining the examination portion corresponding to the examination mode. When the degree of severity of the hyperemia is examined, the control unit 90 determines whether or not the processing of steps S210 to S225 is performed for all the machine learning models 93a to 93f.

When it is not determined in step S225 that the processing is completed for all the models, the control unit 90 repeats the processing of step S210 and the subsequent steps. When it is determined in step S225 that the processing is completed for all the models, the control unit 90 displays the classification results in association with the examination target images so that they can be contrasted (step S230).

That is, the control unit 90 displays the image captured in step S200 on the display unit 70. The control unit 90 displays the classification results acquired in step S220 in association with the images on the display unit 70. There are as many classification results as the number of groups for one machine learning model. For example, when the degrees of hyperemia are classified into the groups 1, 2, 3, and 4 as in the above-mentioned example, four output values obtained by one machine learning model corresponding to the total number of groups are obtained. When the output values are obtained for the six machine learning models 93a to 93f, 4 × 6 = 24 output values are obtained.

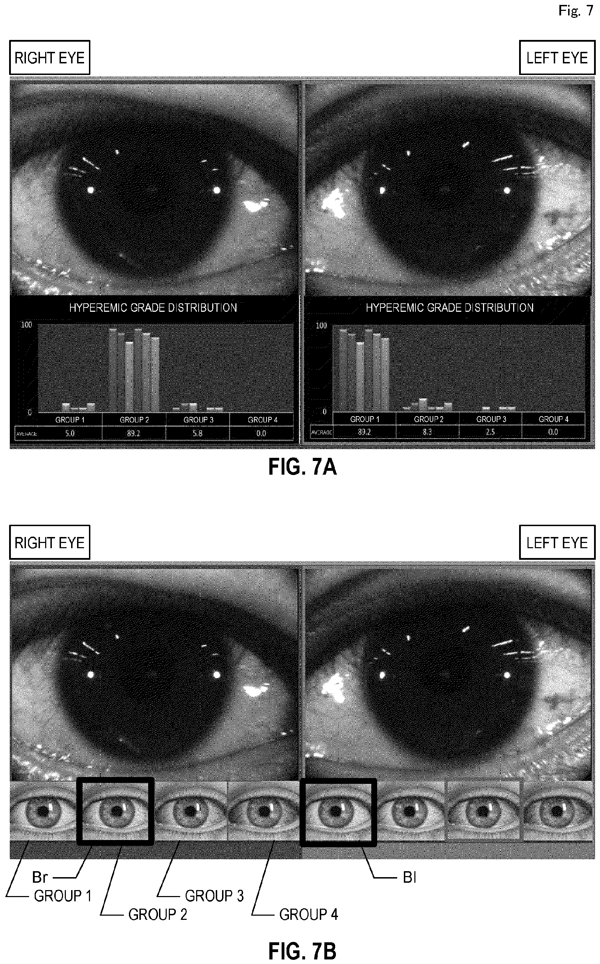

Thus, the control unit 90 displays these output values in a comparable manner for each group, and also displays the output values (probabilities) acquired by the plurality of machine learning models 93a to 93f in a comparable manner for each of the plurality of groups. FIG. 7A is a diagram illustrating a display example. In FIG. 7A, the captured images of the left and right eyes are simultaneously illustrated, and the captured image of the right eye is displayed on the left side and the captured image of the left eye is displayed on the right side.

The output value is illustrated below each captured image. Since the output value according to the present embodiment has a range of 0 to 1 in each of the nodes N₁ to N₄, the control unit 90 converts the output value into % by multiplying the output value by 100. The control unit 90 controls the display unit 70 to draw a display region for each group, and displays the output values of the six machine learning models 93a to 93f in each display region as a bar graph.

That is, in FIG. 7A, characters of the groups 1, 2, 3, and 4 surrounded by rectangles are displayed below the captured images, and the display region of each group is indicated by each rectangle. A bar graph having a length corresponding to the output value (%) indicating the probability of being classified into each group is displayed in each display region. In the example illustrated in FIG. 7A, an average value of the output values is also displayed for each group.

In the example illustrated in FIG. 7A, the average value of the output values corresponding to the group 2 of the right eye illustrated on the left side is the maximum (89.2). Accordingly, the examiner can determine that the degree of severity of the hyperemia is the group 2. The average value of the output values corresponding to the group 1 of the left eye illustrated on the right side is the maximum (89.2). Accordingly, the examiner can determine that the degree of severity of the hyperemia is the group 1.

According to the above configuration, since the outputs of the plurality of machine learning models can be compared, the examiner can determine whether or not the classification result is valid by comparing the outputs within the same group or between different groups. In the present embodiment, since the classification result is obtained by the operation of the machine learning model, it is statistically expected that the accuracy is equal to or greater than the threshold value for determining whether or not the generalization of the machine learning is completed. Accordingly, classification can be performed with this accuracy without depending on subjectivity.

### (4) Other embodiments:

The above embodiment is an example for carrying out the present invention, and various other embodiments can be adopted as long as the state of the examination portion is classified by the machine learning model. For example, the ophthalmic apparatus is not limited to the configuration including the head unit and the main body unit as described above, and may be an ophthalmic apparatus including various other elements. The configuration of the optical system is also an example, and various other optical systems may be adopted.

The examination target image acquisition unit may be able to acquire the examination target image which is the image of the examination portion of the anterior eye portion or a region around the anterior eye portion. That is, the classification of the state of the examination target is obtained based on appearance feature of the anterior eye portion or the examination portion. Thus, the examination target image acquisition unit may be able to acquire the examination target image including the feature.

The examination target is at least one of the examination portion in the anterior eye portion and the examination portion around the anterior eye portion. The examination portion in the anterior eye portion may be the entirety or a part of the anterior eye portion included in the image captured by a camera oriented to the eye to be examined. The examination portion around the anterior eye portion may be a portion present around the anterior eye portion, and may be the entirety or a part of a region around the anterior eye portion included in the image captured by the camera oriented to the eye to be examined. The examination portion may be a normally hidden portion, for example, a portion on the back of the eyelid.

The classification unit may be able to input the examination target image into the machine learning model that classifies the state of the examination portion based on the image of the examination portion and may be able to acquire the classification result. That is, the classification unit may be able to estimate the classification of the examination portion from which the examination target image is acquired based on the machine learning model acquired by machine learning in advance.

The state of the examination portion may be classified into a plurality of predetermined groups. That is, any of the plurality of groups is associated with the image as the teaching data. The classification is performed by inputting the examination target image to the machine learning model acquired by machine learning using the teaching data.

Although the image may be artificially classified when the teaching data is generated in the processing of machine learning to acquire the machine learning model, evaluation criteria for individual classifications are not artificially decided. Accordingly, the decision of the evaluation criteria is not performed depending on subjectivity, and the classification can be performed objectively.

The state may correspond to the examination result of the examination portion, and when the degree of severity of the hyperemia is desired to be examined as described above, for example, the degree of severity of the hyperemia corresponds to the state. When a type classification of a dry eye is desired to be acquired, for example, the type of the dry eye corresponds to the state. In this case, for example, in the above-mentioned ophthalmic apparatus 1, an image of a black eye portion (cornea portion) as the examination portion may be extracted from the captured image and may be classified by machine learning or the machine learning model. For example, the image captured in the examination mode of the staining observation illustrated in FIG. 5A can be used as the captured image.

When a degree of falling of the meibomian gland is desired to be examined, for example, the degree of severity of falling of the meibomian gland corresponds to the state. In this case, for example, in the above-mentioned ophthalmic apparatus 1, an image of the back side of the upper eyelid or the lower eyelid as the examination portion may be extracted from the captured image and may be classified by machine learning or the machine learning model. For example, the image (image captured with infrared rays) captured by the examination mode of the meibomian gland illustrated in FIG. 5A can be used as the captured image.

When the amount of tear is desired to be examined, for example, a height, an area, or the like of the tear meniscus correspond to the state. In this case, for example, in the above-mentioned ophthalmic apparatus 1, an image of the tear meniscus as the examination portion may be extracted from the captured image and may be classified by machine learning or the machine learning model. For example, the image captured by the examination mode of slit capturing illustrated in FIG. 5A can be used as the captured image. As described above, the state can be defined by various elements according to the examination portion, and the state can be defined according to any examination other than these examples.

The input of the examination target image to the machine learning model and the acquisition of the classification result may be in various forms. For example, the examination target image may be enlarged or reduced such that the examination target image can be input to the machine learning model. The examination portion may be enlarged or reduced in order to set the size of the examination portion to be uniform. Of course, image processing such as adjustment of brightness and contrast may be performed. The output of the classification result may be in various forms. That is, the output may be performed in various forms other than the configuration in which the sum of the output values for each of the output nodes is normalized to 1.

Although the configuration in which machine learning is performed by the CNN is adopted in the above-described embodiment, of course, various other methods can be adopted as the machine learning method. Various network structures may be added. The form of the machine learning is not limited. For example, various elements such as the number of layers and nodes constituting the model, the type of the activation function, the type of the loss function, the type of the gradient descent method, the type of the optimization algorithm of the gradient descent method, presence or absence of mini-batch learning, the number of batches, a learning rate, the initial value, the type and the presence or absence of an overlearning suppression method, the presence or absence of a convolution layer, a filter size in the convolution operation, the type of the filter, a type of padding or stride, the type and the presence or absence of the pooling layer, the presence or absence of the fully connected layer, and the presence or absence of a recursive structure may be appropriately selected and machine learning may be performed. Of course, learning may be performed by other machine learning, for example, support vector machine, clustering, reinforcement learning, or the like.

Machine learning in which the structure of the model (for example, the number of layers, the number of nodes per layer, or the like) is automatically optimized may be performed. Machine learning may be performed by various apparatuses, for example, machine learning may be performed by a server. In this configuration, for example, a configuration in which teaching data is collected from a plurality of clients and machine learning is performed on the server based on the teaching data may be adopted.

The display unit may be able to display the classification result. That is, when the classification result can be presented to the examiner, the classification result may be displayed in various forms. The display form of the classification result is not limited to the above-described embodiment, and various forms are assumed. For example, a configuration in which the classification corresponding to the largest value among the estimated values of the classification results is displayed as the classification result and the classification corresponding to the other estimated values is not displayed or the like may be adopted instead of displaying the classification results in a comparable manner.

The display format of classification results is not limited to displaying the output value. For example, an image may be displayed when the classification result corresponding to the largest value among the estimated values of the classification results. As a more specific example, in the above-mentioned ophthalmic apparatus 1, a configuration in which an image indicating a typical example is recorded as the image indicating the state of the examination portion belonging to each of the plurality of groups in the memory 93 in advance can be assumed. In this configuration, the control unit 90 displays the image indicating the typical example when the classification result is displayed.

FIG. 7B is a diagram illustrating an example of such a display. As in FIG. 7A, the captured image of the right eye is displayed on the left side and the captured image of the left eye is displayed on the right side in FIG. 7B. Images indicating typical examples in the groups 1, 2, 3, and 4 are displayed side by side in order from the left below the captured images. The control unit 90 acquires the output values indicating the classification of the examination portion based on the plurality of machine learning models, and considers, for example, the group having the largest average value as the degree of severity of the hyperemia of the examination portion.

When the degree of severity is specified, the control unit 90 selects the image indicating the state of the examination portion belonging to the group corresponding to the classification result from the images indicating the typical examples and emphasizes and displays the selected image. In the example illustrated in FIG. 7B, a frame of the image indicating the typical example is emphasized with a thick line, and thus, the image corresponding to the degree of severity of the hyperemia is illustrated. That is, in the example illustrated in FIG. 7B, the degree of severity of the hyperemia of the right eye illustrated on the left side is assumed to be the group 2, and a second image Br from the left is emphasized. Accordingly, the examiner can determine that the degree of severity of the hyperemia is the group 2. The degree of severity of the hyperemia of the left eye illustrated on the right side is assumed to be the group 1, and a first image Bl from the left is emphasized. Accordingly, the examiner can determine that the degree of severity of the hyperemia is the group 1. According to the above configuration, the examiner can intuitively recognize the classification result and the degree of severity of the hyperemia in association with each other.

The display form of the classification result is not limited to the above-mentioned form. For example, the number of groups is not limited to four, and may be less or more. Even though the number of groups is a predetermined number, the number of groups may be displayed such that the degree of severity changes continuously at the display of classification. For example, the probabilities for representative degrees of severity may be acquired based on FIG. 7A and the classification results of a larger number of groups, and the probabilities are acquired and displayed such that the degrees of severity for which numerical values of the classification results are not obtained are continuously changed. FIG. 6B illustrates an example of such a display.

The method for classifying the state of the examination portion by the machine learning model can be applied as an invention of a method or a program. The ophthalmic apparatus, method, and program described above can be assumed to be achieved as a single apparatus or as a part of an apparatus having a plurality of functions, and include various forms.

### Reference Signs List

- 10: first light projection optical system
- 10a: first light source
- 10b: lens
- 10c: light adjustment member
- 10d: lens
- 11a: light source
- 11b: light source
- 20: second light projection optical system
- 20a: second light source
- 20b: lens
- 20c: light adjustment member
- 20d: lens
- 21a: light source
- 21b: light source
- 30: light receiving optical system
- 30a: image sensor
- 30b: lens
- 30c: light adjustment member
- 30d: lens
- 32c: light receiving adjustment unit
- 40: X-Y alignment optical system
- 40a: X-Y alignment light source
- 40b: lens
- 40c: half mirror
- 50: Z alignment optical system
- 50a: Z alignment light source
- 50b: lens
- 50c: lens
- 50d: position detection sensor
- 60: visual fixation optical system
- 60a: visual fixation light source
- 60b: visual fixation target
- 60c: lens
- 60d: half mirror
- 70: display unit
- 80: touch panel
- 90: control unit
- 90a: examination target image acquisition module
- 90b: classification module
- 91: XYZ drive control unit
- 92: joystick
- 93: memory
- 93a: machine learning model
- 95: adjustment unit drive unit
- 100: machine learning system
- 200: control unit
- 210: machine learning program
- 210a: machine learning module
- 300: recording medium
- 300a: teaching data
- 400: user I/F unit

## Claims

1. An examination target classification system comprising:
an examination target image acquisition unit that acquires an examination target image which is an image of an examination portion of an anterior eye portion or a region around the anterior eye portion;
a classification unit that inputs the examination target image to a machine learning model which classifies a state of the examination portion based on the image of the examination portion, and acquires a classification result; and
a display unit that displays the classification result.

2. The examination target classification system according to claim 1, **characterized in that**
the classification unit acquires the classification result by each of a plurality of the machine learning models, and
the display unit displays the classification result in each of the plurality of machine learning models in a comparable manner.

3. The examination target classification system according to claim 2, **characterized in that**
the state of the examination portion is classified into any of a plurality of predetermined groups,
the classification unit acquires a probability that the state of the examination portion belongs to the group for each of the plurality of groups based on each of the plurality of machine learning models, and
the display unit displays the probability acquired for each of the plurality of machine learning models for each of the plurality of groups in a comparable manner.

4. The examination target classification system according to claim 1 or 2, **characterized in that**
the state of the examination portion is classified into any of a plurality of predetermined groups, and
the display unit selects and displays an image indicating the state of the examination portion belonging to the group corresponding to the classification result from among images prepared as the image indicating the state of the examination portion belonging to each of the plurality of groups.

5. The examination target classification system according to any one of claims 1 to 4, **characterized in that**
the machine learning model is acquired by machine learning based on teaching data in which an image obtained by removing a portion other than the examination portion from an image including the examination portion is associated with the classification result of the state of the examination portion, and
the examination target image is acquired by removing the portion other than the examination portion from an image obtained by capturing the examination portion.

6. An examination target classification method comprising:
an examination target image acquisition step of acquiring an examination target image which is an image of an examination portion of an anterior eye portion or a region around the anterior eye portion;
a classification step of inputting the examination target image to a machine learning model which classifies a state of the examination portion based on the image of the examination portion, and acquires a classification result; and
a display step of displaying the classification result.

7. An examination target classification program causing a computer to function as:
an examination target image acquisition unit that acquires an examination target image which is an image of an examination portion of an anterior eye portion or a region around the anterior eye portion;
a classification unit that inputs the examination target image to a machine learning model which classifies a state of the examination portion based on the image of the examination portion, and acquires a classification result; and
a display unit that displays the classification result.
